# EUROPEAN PATENT APPLICATION

(11) **EP 4 667 899 A1**
(43) Date of publication of application: **24.12.2025**
(21) Application number: 24183112.2
(22) Date of filing: 19.06.2024
(51) Int. Cl.: G01N 1/10, G01N 1/38, G01N 33/18, G01N 1/40

(54) **METHOD OF OBTAINING LIQUID SAMPLE COMPRISING A TRACE ANALYTE FROM A CONTAINER AND DEVICE FOR PROCESSING SAID LIQUID SAMPLE**

(71) Applicant: Biotage AB, 751 03 Uppsala (SE)
(72) Inventor: LUNDGREN, Pär, 753 17 UPPSALA (SE); WESSMAN, Markus, 754 18 UPPSALA (SE)
(74) Representative: Brann AB

(57) **Abstract**

Examples of the present disclosure may include a system and method of obtaining liquid sample comprising a trace analyte from a container, the method comprising: receiving the container comprising the liquid sample; pumping the liquid sample from the container by a hollow fluid conveying member having a first end submerged in the sample and a second end in fluid communication with a pump, wherein an unavoidable remainder of the liquid sample remains in the container; after the pumping the liquid sample from the container, providing an aerosol of a solvent in the container using an aerosol spray device provided inside the container and connected to a solvent source, such that the aerosol wets an inner surface of the container and a mixture of the solvent and remainder of liquid sample is formed in the container; pumping the mixture from the container through the hollow fluid conveying member using the pump towards an outlet; and processing the pumped liquid sample and the pumped mixture.

## Description

The present patent disclosure relates to the field of processing liquid sample comprising a trace analyte. Some examples of the present patent disclosure relate to obtaining said liquid sample from container and extracting said trace analyte from the liquid sample.

In general, obtaining liquid samples comprising trace analytes from containers involves techniques such as rinsing, which requires a significant amount of liquid to be effective. While this approach is widely used, it has several disadvantages. Firstly, the amount of liquid required can be problematic for some testing techniques, particularly those that require very small volumes of sample. Secondly, for certain types of trace analytes, such as fluorinated chemicals (e.g. PFAS), for example in drinking water samples, there are strict regulations governing how much the testing solution can be diluted. Finally, processing waste containing these chemicals can be expensive, making it a significant cost factor.

It is desirable to obtain as much trace analyte as possible from a container in various fields such as environmental monitoring, medical testing, and food safety analysis. However, traditional methods often struggle to meet this goal due to limitations in sample handling, storage, and processing. In light of these challenges, there is a need for improved techniques that can efficiently extract trace analytes from containers while minimizing waste and cost.

It is an object, among objects, to provide an improved method of obtaining liquid sample comprising a trace analyte from a container.

There is provided, in accordance with a first aspect, a method of obtaining liquid sample comprising a trace analyte from a container, the method comprising receiving the container comprising the liquid sample; pumping the liquid sample from the container by a hollow fluid conveying member having a first end submerged in the sample and a second end in fluid communication with a pump, wherein an unavoidable remainder of the liquid sample remains in the container; after the pumping the liquid sample from the container, providing an aerosol of a solvent in the container using an aerosol spray device provided inside the container and connected to a solvent source, such that the aerosol wets an inner surface of the container and a mixture of the solvent and remainder of liquid sample is formed in the container; pumping the mixture from the container through the hollow fluid conveying member using the pump towards an outlet; and processing the pumped liquid sample and the pumped mixture.

Beneficially, by using the aerosol spray device, only a limited amount of solvent is required to obtain at least similar, or even better, results than manual rinsing, which is considered to be the best reference technique. The amount of solvent required to obtain a similar or better result is lower than 15 ml for a 1L container. When using less solvent, the present method is even better than the reference technique of manual rinsing. In addition, the method allows for automated rinsing of the sample containers.

In the present patent disclosure, the term "trace analyte" is used to indicate an analyte, or chemical substance to be tested or analysed, wherein the chemical substance is present in the sample liquid at a concentration below 1 part-per-million (ppm).

The unit part-per-million may refer to the mass of the analyte per volume of liquid sample. For example, 1 ppm would then be equivalent to 1 µg analyte per ml of liquid sample. Further examples include: 1 ng of analyte per ml of liquid sample would be equivalent to 1 part-per-billion (ppb); 1 pg (picogram) of analyte per ml of liquid sample is equivalent to 1 part-per-trillion (ppt); and 1 fg (femtogram) of analyte per ml of liquid sample is equivalent to 1 part-per-quadrillion (ppq). When the analyte is present in such low concentrations, the method beneficially enables in increased amount of analyte to be recovered from the sample container. The analyte may then be concentrated using, for example, solid phase extraction suitable for the analyte. The choice of suitable solid phase extraction is known in the art.

According to some examples, the liquid sample comprises the trace analyte at a concentration below 100 (ppm). Example concentrations include 10 ppm, 1 ppm, 0.1 ppm (or 100 parts-per-billion (ppb), 0.01 ppm (or 10 ppb). According to some examples, the liquid sample comprises the analyte at a concentration below 1 part-per-billion, ppb.

According to some examples, the trace analyte is a per- or polyfluoroalkyl substance (PFAS).

According to some examples, the aerosol spray device is configured to wet an inner circumferential part or section of an inner surface of the container. The inner circumferential section may be at a certain height within the container. Droplets may be formed on the inner circumferential part, which then trickle down to wet the remainder of the inner surface of the container below the inner circumferential section.

According to some examples, the aerosol spray device comprises a nozzle configured to output an aerosol in all directions surrounding the nozzle. The nozzle may comprise a nozzle centre directed towards the base of the container. The nozzle may have a spray angle, for example in the range of 90° - 150°. The nozzle beneficially wets at least a circumferential section of the inner surface of the container. The nozzle may be a full cone nozzle, a misting nozzle, or a hollow cone nozzle. A full cone nozzle beneficially directly wets the inner surface of the container that that nozzle is directed towards.

According to some examples, the aerosol spray device comprises a hollow cone nozzle. A hollow cone nozzle beneficially wets the surface with small droplets such that at least a circumferential section of the inner surface of the container may be wetted in a highly uniform manner.

According to some examples, the aerosol is provided such that the wetted inner surface includes inner surface of the container that has been in contact with the liquid sample.

According to some examples, the aerosol is provided such that the wetted inner surface includes substantially all of an inner surface of the container that has been in contact with the liquid sample. The wetting may be comprise indirect wetting, indirect meaning that the wetting is caused by fluid flowing or trickling down the inner surface of the container.

"Substantially all of the inner surface" may indicate at least 90% of the inner surface that has been in contact with the liquid sample, such as 95%, or 98%.

According to some examples, at most 20 ml of solvent is used to provide the aerosol.

According to some examples, at most 10 ml of solvent is used to provide the aerosol.

According to some examples, at least 2 ml of solvent is used to provide the aerosol.

According to some examples, a volume of solvent in a range of 2 to 20 ml is used to provide the aerosol. The range may be 2 to 10 ml of solvent. The amount of solvent used may be proportional to an inner surface area of the container.

According to some examples, the outlet is in fluid connection with a processing device configured to process the liquid sample and the mixture, wherein the processing comprises providing the pumped liquid sample and the mixture to the treatment device.

According to some examples, the processing device is an extraction device configured to extract the trace analyte from the liquid sample.

According to some examples, the extraction device is a solid-phase extraction, SPE, device. After the analyte is extracted with SPE, the analyte can be taken to a further device for analysis or quantification, such as with Liquid Chromatography (LC) combined with Mass Spectroscopy (MS) or other methods, for quantization or identification or both. As an example, the present method and device, when using SPE to extract the analyte, and thereafter determining the amount of analyte using UPLC-TQ/MS (Ultra-Performance Liquid Chromatography-Triple Quadruple Mass Spectroscopy), concentrations as low as 0.2 ppt can be determined for PFAS analytes. This 0.2 ppt is a thousand times lower than the Limit of Quantitation (LOQ) specified in Draft Method 1633 (August 2021) of the U.S. Environmental Protection Agency for analysis of PFAS in Aqueous, Solid, Biosolids, and Tissue Samples by LC-MS/MS. It is also lower than the LCMRLs (Lowest Concentration Minimum Reporting Level) for all listed PFAS of both Method 533 (Table 7) and Method 537.1 (Table 5) for determination of PFAS in drinking water by solid phase extraction and liquid chromatography/tandem mass spectrometry (LC/MS/MS) of the U.S. Environmental Protection Agency.

When the analyte is PFAS, the SPE may be provided with a suitable SPE cartridge. One example of such a suitable SPE cartridge is a cartridge containing styrenedivinyl-benzene (SDVB) polymeric sorbent phase.

According to some examples, the pump is a syringe pump.

According to some examples, the method comprises flushing a flow path in a direction from the syringe pump to the outlet with nitrogen, wherein the nitrogen is introduced near an outlet of the syringe pump, preferably within 2 cm from the outlet of the syringe pump.

According to some examples, the hollow fluid conveyor is a conduit, such as a pipe or a tube.

According to some examples, the liquid sample is an aqueous liquid sample.

According to some examples, the processing comprises processing at least 90% of the trace element present in the container.

According to some examples, the method is performed by a controller of a device for processing the liquid sample.

According to a second aspect, there is provided a device for processing a liquid sample comprising a trace analyte from a sample container, the device comprising: a pump; a hollow fluid conveying member configured to be positioned within the sample container with a first end thereof and in fluid communication with the pump with a second end thereof; an aerosol spray device configured to be provided inside the container and in fluid communication with a solvent source; and an outlet in fluid communication with the pump, wherein the device is configured to: pump the liquid sample from the container via the hollow fluid conveying member, wherein, when the liquid sample is pumped, an unavoidable remainder of the liquid sample remains in the container; after the pumping the liquid sample from the container, provide an aerosol of a solvent in the container using the aerosol spray device provided inside the container and connected to the solvent source, such that the aerosol wets an inner surface of the container and a mixture of the solvent and remainder of liquid sample is formed in the container; and pump the mixture from the container through the hollow fluid conveying member using the pump towards the outlet.

According to some examples, the device is a solid phase extraction device comprising a solid phase extraction section.

According to some examples, the pump is a syringe pump and comprising a pump outlet, wherein the device comprises a gas valve configured to be connected to a gas source.

According to some examples, the device is configured to flush a flow path from the syringe pump to the outlet with gas from the gas source, such as nitrogen, wherein the device is arranged such that the gas is introduced near a pump outlet of the syringe pump, preferably within 2 cm from the pump outlet of the syringe pump. In this way, the amount of trace analyte that is obtained and processed is increased even further, since almost all of the fluid path is flushed with the gas, such as nitrogen. The gas may be an inert gas or gas mixture. It is additionally found that, if the gas is introduced within 2 cm from the pump outlet, even at least a part of any liquid remaining between the gas inlet and the pump outlet (in the 2 cm) also follows along with the gas.

According to some examples, the device comprises a controller configured to control at least the pumping of the liquid sample, the providing of an aerosol, and the pumping of the mixture.

The device may comprise polypropylene material for all elements that are in contact with the liquid sample, such as polypropylene tubing. This is advantageous when the analyte is, for example, a PFAS.

According to some examples, the controller is further configured input the aerosol spray device into the container. The device may comprise one or more motors and/or actuators for performing various movements, such as inputting the aerosol spray device into the container and/or actuating the pump(s), such as the syringe pump(s), and/or actuating the valve(s).

It will be understood that technical advantages and effects associated with features and/or examples of one aspect, apply to the corresponding, similar or equivalent features and/or examples of the other aspects. It will also be apparent that the features of the various aspects and/or examples thereof may be applied to the other aspects and/or examples thereof.

### Brief Description of the Drawings

The accompanying drawings are used to illustrate presently preferred non-limiting examples of devices of the present disclosure. The above and other advantages of the features and objects of the disclosure will become more apparent, and the aspects and examples will be better understood from the following detailed description when read in conjunction with the accompanying drawings, in which:
FIG. 1 is a flow chart of a method according to some examples of the present disclosure;
FIG. 2 is a schematic drawing of a device for processing a liquid sample according to some examples of the present disclosure;
FIG. 3 is a schematic drawing of a device for processing a liquid sample according to some examples of the present disclosure; and
FIG. 4 is a schematic drawing of a device for processing a liquid sample according to some examples of the present disclosure.

### Detailed Description

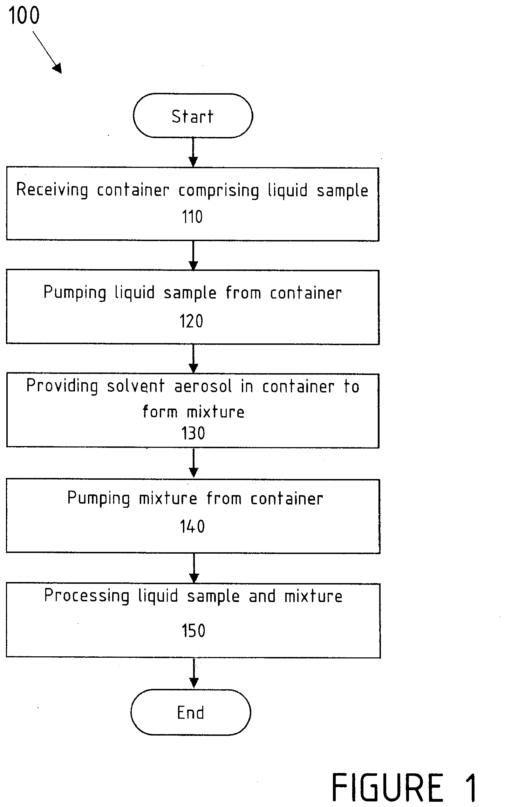

Referring to Figure 1, there is provided a method 100 of obtaining liquid sample comprising a trace analyte from a container. The method 100 comprises receiving 110 the container comprising the liquid sample. The method 100 further comprises pumping 120 the liquid sample from the container by a hollow fluid conveying member having a first end submerged in the sample and a second end in fluid communication with a pump. An unavoidable remainder of the liquid sample remains in the container. This unavoidable remainder may, for example, be present on a lower end of the container and/or on an inner surface of the container.

The method 100 comprises, after the pumping the liquid sample from the container, providing 130 an aerosol of a solvent in the container using an aerosol spray device provided inside the container and connected to a solvent source. The aerosol spray device provides the aerosol such that the aerosol wets an inner surface of the container. A mixture of the solvent and remainder of liquid sample is formed in the container. The aerosol may be provided such that the wetted inner surface includes inner surface of the container that has been in contact with the liquid sample.

The method 100 comprises pumping 140 the mixture from the container through the hollow fluid conveying member using the pump towards an outlet. The liquid sample pumped in pumping step 120 and the mixture pumped in pumping step 140 may both be processed separately or together.

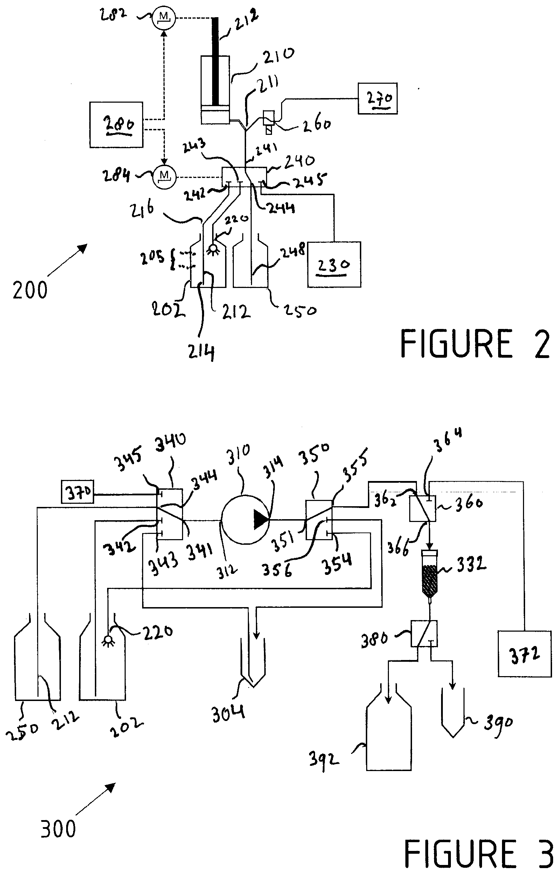

In some examples, the outlet is in fluid connection with a processing device configured to process the liquid sample and the mixture, wherein the processing comprises providing the pumped liquid sample and the mixture to the treatment device. The processing device may be an extraction device configured to extract the trace analyte from the liquid sample. An example of an extraction device is a solid-phase extraction (SPE) device, as shown in relation to Figures 2-4.

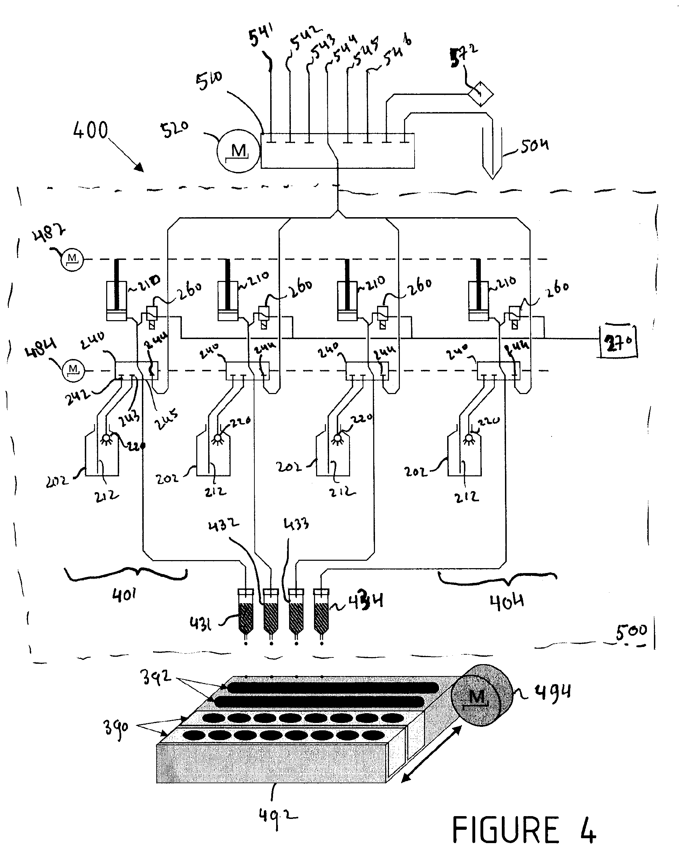

The method 100 may comprise flushing a flow path from the syringe pump to the outlet with nitrogen, wherein the nitrogen is introduced near an outlet of the syringe pump. In some examples, the nitrogen is introduced within 2 cm from the outlet of the syringe pump. This is explained in further detail in relation to Figure 4.

In Figure 2 there is shown a device 200 for processing a liquid sample comprising a trace analyte from a sample container 202. The device 200 comprises a syringe pump 210. The syringe pump 210 is one example of a pump.

The device 200 further comprises a hollow fluid conveying member 212 configured to be positioned within the sample container 202 with a first end 214 thereof and in fluid communication with the pump 210 with a second end 216 thereof. The hollow fluid conveying member may be implemented as a conduit, such as a pipe or a tube.

The device 200 comprises an aerosol spray device 220 configured to be provided inside the container 202. The aerosol spray device 220 is, in this embodiment, in fluid communication with a solvent source 248 or solvent inlet 248. A suitable solvent held by solvent container 250 may be in fluid communication with the solvent inlet 248. Although one solvent container 250 and corresponding inlet 248 are drawn, there may be multiple solvent inlets provided in the device 200, each for a separate container so that multiple solvents can be chosen from. The solvent inlet 248 is, in this example, embodied as a tube or pipe. The device comprises outlet 230, which is in fluid communication with the pump 210.

The solvent inlet 230 is in fluid communication with the aerosol spray device 220 via a valve 240. The valve 240 may comprise a plurality of ports arranged to selectively bring the pump 210 in fluid communication with the sample container 202, the aerosol spray device 220, the solvent inlet 248, or the outlet 230. The plurality of ports in this example comprises a first port 241, a second port 242, a third port 243, a fourth port 244 and a fifth port 245. The first port 241 is arranged to receive and provide fluid from and respectively to the pump 210. The second port 243 is configured to receive fluid from the sample hollow conveying member 212. The third port 243 is configured to provide liquid to the aerosol spray head 220. The fourth port 244 is configured to receive solvent from the solvent inlet 248. The outlet 230 is, in this example, in fluid communication with the pump 210 via the fifth port 245 of the valve 240. The plurality of valve ports may comprise additional ports in case there are, for instance, multiple solvent inlets provided in the device 200 as mentioned above.

The device 200 may comprise a gas valve 260, for instance to let in nitrogen as a purging gas. In this example, the nitrogen is introduced close to the outlet port 211 of the pump 210, such as within 2 cm of the outlet port 211 of the pump 210. In this way, as much analyte as possible can be flushed out towards the outlet 230. The valve is in fluid communication with a gas inlet 270. This gas inlet 270 may receive pressurized nitrogen or another inert gas, or air.

The device 200 is configured to pump the liquid sample from the container via the hollow fluid conveying member 212. The valve 240 is then in a state where there is a flow path from the first port 241 to the second port 242. The liquid sample fills the pump 210. An unavoidable remainder of the liquid sample remains in the sample container 202 after pumping. The valve 240 is switched to connect the first port 241 to the fifth port 245 and the pumped liquid sample is pumped towards the outlet 230. Optionally, the outlet 230 may provide the liquid to a solid phase extraction device. A nitrogen purge may be performed optionally to increase the amount of sample fluid reaching the outlet 230.

After the pumping the liquid sample to the outlet 230, the valve 240 is switched to connect the first port 241 to the fourth port 244, such that the pump 210 is able to pump in solvent, such as 6 ml or 10 ml of solvent. Thereafter the valve 240 is switched to connect the first port 241 to the third port 243, such that the pumped solvent can then be provided to the aerosol spray device 220.

The aerosol spray device 220 is arranged such that the aerosol wets an inner surface of the container 202 and a mixture of the solvent and remainder of liquid sample is formed in the container 202. Then, the valve 240 is switched to again connect first port 241 to second port 242, such that the mixture can be pumped from the container 202 through the hollow fluid conveying member 212 towards the outlet 230, by appropriately switching the valve 240 and using the pump 210 as described above. A nitrogen purge may now be done again.

The device 200 comprises a controller 280 configured to control the pumping of the liquid sample, the providing of an aerosol, and the pumping of the mixture. The controller 280 is to these ends configured to control the pump action of the pump 210. In Figure 2, the controller 280 is configured to control a first stepper motor 282 which is configured to move the plunger 212 of the syringe pump 210. The controller 280 is also configured to control the switching between ports of the valve 240 described above. In Figure 2, the controller 280 is configured to control a second stepper motor 284 which is configured to switch the flow path within the valve 240 between the first valve port 241 and any one of the second 242, third 243, fourth 244, and fifth 245 valve ports.

According to some examples, the aerosol spray device 220 is configured to wet an inner circumferential part or section 205 of an inner surface of the container 202. The inner circumferential section may be at a certain height within the container. Droplets may be formed on the inner circumferential part, which then flow or trickle down to wet the remainder of the inner surface of the container below the inner circumferential section. Alternatively, the entire inner wall, for example below a certain height, may be wetted by the aerosol spray device 220 directly.

According to some examples, the aerosol spray device 220 comprises a nozzle configured to output an aerosol in all directions surrounding the nozzle. The nozzle may comprise a nozzle centre directed towards the base of the container. The nozzle may have a spray angle, for example in the range of 90° - 150°. The nozzle beneficially wets at least a circumferential section of the inner surface of the container. The nozzle may be a full cone nozzle, a misting nozzle, or a hollow cone nozzle. A full cone nozzle beneficially directly wets the inner surface of the container that that nozzle is directed towards.

According to some examples, the aerosol spray device 220 comprises a hollow cone nozzle. A hollow cone nozzle beneficially wets the surface with small droplets such that at least a circumferential section of the inner surface of the container may be wetted in a highly uniform manner.

According to some examples, the aerosol is provided such that the wetted inner surface includes inner surface of the container 202 that has been in contact with the liquid sample.

According to some examples, the aerosol is provided such that the wetted inner surface includes substantially all of an inner surface of the container that has been in contact with the liquid sample. The wetting may comprise indirect wetting, indirect meaning that the wetting is caused by fluid flowing or trickling down the inner surface of the container.

"Substantially all of the inner surface" may indicate at least 90% of the inner surface that has been in contact with the liquid sample, such as 95%, or 98%.

In the example of Figure 2, a syringe pump 210 is used. Since a syringe pump has a single port 211 for input and output, the valve 240 is associated with the syringe pump 210. The use of other types of pumps with separate ports for input and output allow one pump to be used with multiple valves and/or multiple sample containers and processing devices, as described, for example, in relation to Figure 3.

In Figure 3, there is shown an example of a device according to the present patent disclosure. The device 300 works in a similar manner as device 200, except as described below. The pump 310 is not a syringe pump in this case, and comprises a pump inlet 312 and a pump outlet 314. With this pump 310, fluid always flows from the pump inlet 312 towards the pump outlet 314.

To accommodate this different choice of pump, the device 300 comprises a first switchable valve 340 at an inlet side of the pump 310 and a second switchable valve 350 at an outlet side of the pump 310. The first switchable valve 340 comprises a first plurality of switchable valve ports. The first plurality of switchable valve ports comprises individual outlet port 341, and a first 342, second 343, third 344 and fourth 345 inlet ports. The fourth inlet port 345 is connected to a first gas inlet 370. The second switchable valve 350 comprises a second plurality of switchable valve ports. The second plurality of switchable valve ports comprises an individual inlet port 351, and a first 354, second 355, and third 356 outlet port. The device 300 further comprises a third switchable valve 360 and a fourth switchable valve 380. The third switchable valve 360 is configured to switch from an analysis fluid inlet port 362 to a purge gas inlet port 364, which is connected to second gas inlet 372. The fourth switchable valve 380 is with its input side in fluid communication with the solid phase extraction (SPE) device 332 and allows to select where the output of the SPE device 332 is directed: Either to a waste container 392 or a vial 390 which can be used for further analysis.

The device 300 is configured to pump the liquid sample from the container 202 via the hollow fluid conveying member 212. The valve 340 is then in a state where there is a flow path from the first inlet port 342 to the outlet port 341. The second switchable valve 350 is switched such that the inlet port 351 is connected to the second outlet port 355, so that the fluid can flow towards the SPE device 332. An unavoidable remainder of the liquid sample remains in the sample container 202 after pumping. The valve 340 is switched appropriately to connect the solvent inlet 212 to the pump. The solvent can be pumped directly to the aerosol spray device 220 by appropriately switching the valve 350, or can be mixed in mixing chamber 304. The device 200 can also be equipped with a mixing chamber similar to mixing chamber 304, and valve 240 may comprise an additional valve port to connect to the mixing chamber.

A controller is not indicated in Figure 3, but may also be provide for, similar to device 200, to control the pump and valves, for example.

In the devices 200 and 300, there may be provided multiple pumps with respective valves, each for handling fluid from a respective sample container. Each pump/valve(s) combination may be called a channel. The devices 200 and/or 300 may thus comprise multiple channels, such as four or eight channels. The solvent inlet 212, the mixing chamber 304, the waste container 392 and gas sources 370 and 372 may be shared by the channels.

An example device 400 with multiple channels is shown in Figure 4. A first bank 500 comprises four channels. A first channel 401 is indicated, which also comprises the first SPE device 431, and a fourth channel 404 is indicated, which also comprises the fourth SPE device 434. A second channel can be seen next to the first channel 401, wherein the second channel also comprises the second SPE device 432. A third channel is visible next to the fourth channel 404, wherein the third channel comprises the third SPE device 433.

Each channel comprises its own pump 210, valve 240, fluid inlet 212, and the like, similar to the device 200 of Figure 2. The channels share, however, the solvent valve 510 which is configured to switch the bank 500 to any one of first to sixth solvent inlets 541-546, second gas inlet 572, and mixing chamber 504, which are all shared by the various channels. The device 400 may comprise an additional bank (not shown) comprises fifth to eight channels. The second bank may share the same solvent inlets 541-546, second air inlet 572, and mixing chamber 504. The selection between solvent, air inlet or mixing chamber is done in this example by control of third stepper motor 520, configured to control the switching of valve 510. The valves may be implemented as rotary selector valves.

A slide mechanism 492 supports the two sets of elution vials 390 and the channels for the two waste streams 392. The slide mechanism 492 positions the appropriate vial or channel under the respective output nozzles of the columns 431-434 using the stepper motor 394.

An extraction method may comprise a series of steps. There may be two types of steps: Pump steps and Wait steps. A Pump step transfers a specified amount of fluid from a specified input to a specified out, optionally at a specified flow rate. A Wait step delays for a specified time, with the option of turning on the purge (nitrogen) gas during the delay time.

The devices described herein may be controlled via a user input device (e.g. a touch screen) connected to the device, allowing the user to control the instrument. Optionally, a separate computing device may be connected to the instrument, allowing the user to control the instrument with a keyboard, mouse, and external monitor. This PC may be used to download run reports, upload extraction methods and firmware upgrades, transfer data over a computer network, and provide additional features such as multi-language support and advanced method editing.

### Test examples

To test the performance of the present method and device in obtaining as much sample liquid/analyte from the sample container using the aerosol spray head, the following was performed.

5 mg of a dye was mixed with 250ml methanol to form a dye solution. 1 ml of this dye solution was added to either 250 ml or 1000 ml water to form test solutions.

A spectrometer set to a detection wavelength of 490 nm, suitable for the dye was setup to measure samples with the dye solution. The light absorbance and transmission were recorded. The spectrometer was set to 0 absorbance and 100% transmission for a sample obtained by rinsing a sample bottle by hand with the same volume of solvent (i.e. 5 ml or 15 ml as in the Table 1 below), considered to be the gold standard of obtaining liquid sample from a container.

Then the method was performed on the bottles with test solutions. This comprises the step of pumping the solution from the test solution bottle and thereafter spraying solvent with the aerosol spray head. The mixture comprising the dye and the solvent introduced via the aerosol spray head was then measured with the spectrometer relative to the reference. The results are indicated in the table below.

**Table 1: results of rinsing test with dye solution using aerosol.**

| Sample bottle volume (ml) | Solvent amount (ml) | ABS | T |
|---|---|---|---|
| 250 | 5 | -0.001 | 100.1 |
| 250 | 5 | 0.006 | 98.6 |
| 1000 | 5 | 0.013 | 97 |
| 1000 | 5 | 0.034 | 92.4 |
| 1000 | 15 | -0.019 | 104.4 |
| 1000 | 15 | -0.011 | 102.5 |

In general, the results show that the absorbance remains close to 0 and the transmission close to 100%, which indicates that the obtaining of sample from the containers using the method similar results as the manual rinsing, even when only 5 ml of solvent is used, and also for the larger 1000 ml sample bottle.

When the solvent amount was 5 ml, the aerosol rinsing performed better than the rinsing by hand, given the generally higher absorbance and lower transmission values (2^{nd} to 4^{th} rows in Table 1). Since these values are relative to the respective reference values recorded for rinsing by hand with the same amount of solvent of 5 ml, a higher absorption and lower transmission indicates that more of the dye is present in the sample.

Although the present invention has been described with reference to specific examples, also shown in the appended drawings, it will be apparent to those skilled in the art that many variations and modifications can be done within the scope of the present patent disclosure as described in the specification and defined with reference to the claims below.

## Claims

1. Method of obtaining liquid sample comprising a trace analyte from a container, the method comprising:
receiving the container comprising the liquid sample;
pumping the liquid sample from the container by a hollow fluid conveying member having a first end submerged in the sample and a second end in fluid communication with a pump, wherein an unavoidable remainder of the liquid sample remains in the container;
after the pumping the liquid sample from the container, providing an aerosol of a solvent in the container using an aerosol spray device provided inside the container and connected to a solvent source, such that the aerosol wets an inner surface of the container, and a mixture of the solvent and remainder of liquid sample is formed in the container;
pumping the mixture from the container through the hollow fluid conveying member using the pump towards an outlet; and
processing the pumped liquid sample and the pumped mixture.

2. Method according to claim 1, wherein the liquid sample comprises the trace analyte at a concentration below 100 parts-per-billion, ppb.

3. Method according to claim 2, wherein the liquid sample comprises the analyte at a concentration below 1 part-per-billion, ppb.

4. Method according to claim 2 or 3, wherein the trace analyte is a per- or polyfluoroalkyl substance, PFAS.

5. Method according to any one the preceding claims, wherein the aerosol spray device comprises a hollow cone nozzle.

6. Method according to any one of the preceding claims, wherein the aerosol is provided such that the wetted inner surface includes inner surface of the container that has been in contact with the liquid sample.

7. Method according to any one of the preceding claims, wherein
at most 20 ml of solvent is used to provide the aerosol; or
at most 10 ml of solvent is used to provide the aerosol.

8. Method according to any one of the preceding claims, wherein the outlet is in fluid connection with a processing device configured to process the liquid sample and the mixture, wherein the processing comprises providing the pumped liquid sample and the mixture to the treatment device.

9. Method according to claim 8, wherein the processing device is an extraction device configured to extract the trace analyte from the liquid sample.

10. Method according to claim 9, wherein the extraction device is a solid-phase extraction, SPE, device.

11. Method according to any one of the preceding claims, wherein
the pump is a syringe pump, and optionally further comprising flushing a flow path from the syringe pump to the outlet with nitrogen, wherein the nitrogen is introduced near an outlet of the syringe pump, preferably within 2 cm from the outlet of the syringe pump; and/or
the hollow fluid conveyor is a conduit, such as a pipe or a tube; and/or
the liquid sample is an aqueous liquid sample; and/or
the processing comprises processing at least 90% of the trace element present in the container.

12. Method according to any one of the preceding claims, wherein the method is performed by a controller of a device for processing the liquid sample.

13. Device for processing a liquid sample comprising atrace analyte from a sample container, the device comprising:
a pump;
a hollow fluid conveying member configured to be positioned within the sample container with a first end thereof and in fluid communication with the pump with a second end thereof;
an aerosol spray device configured to be provided inside the container and in fluid communication with a solvent source; and
an outlet in fluid communication with the pump,
wherein the device is configured to:
pump the liquid sample from the container via the hollow fluid conveying member, wherein, when the liquid sample is pumped, an unavoidable remainder of the liquid sample remains in the container;
after the pumping the liquid sample from the container, provide an aerosol of a solvent in the container using the aerosol spray device provided inside the container and connected to the solvent source, such that the aerosol wets an inner surface of the container and a mixture of the solvent and remainder of liquid sample is formed in the container; and
pump the mixture from the container through the hollow fluid conveying member using the pump towards the outlet.

14. Device according to claim 13, wherein
the device is a solid phase extraction device comprising a solid phase extraction section; and/or
the pump is a syringe pump and comprising a pump outlet, wherein the device comprises a gas valve configured to be connected to a nitrogen source, wherein the device is configured to flush a flow path from the syringe pump to the outlet with nitrogen, wherein the device is arranged such that the nitrogen is introduced near a pump outlet of the syringe pump, preferably within 2 cm from the pump outlet of the syringe pump.

15. Device according to claim 13 or 14, comprising a controller configured to control the pumping of the liquid sample, the providing of an aerosol, and the pumping of the mixture.
